# EUROPEAN PATENT APPLICATION

(11) **EP 0 760 239 A2**
(43) Date of publication of application: **05.03.1997**
(21) Application number: 96306254.2
(22) Date of filing: 29.08.1996
(51) Int. Cl.: A61K 31/40, A61K 31/415

(54) **Therapeutic agents for use in cancer therapy**

(30) Priority: 01.09.1995 US 3136
(71) Applicant: PFIZER INC., New York, N.Y. 10017 (US)
(72) Inventor: Kajiji, Shama M., Mystic, Connecticut 06355 (US); Lyssikatos, Joseph P., Gales Ferry, Connecticut 06335 (US)
(74) Representative: Ruddock, Keith Stephen

(57) **Abstract**

This invention relates to the use of compounds of the formulae I,IIA and IIB below for manufacturing medicaments for the treatment or prevention of cancer, 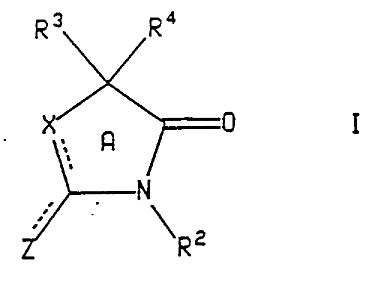 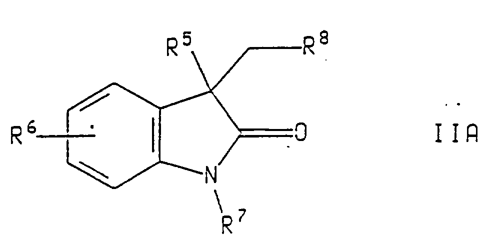 or 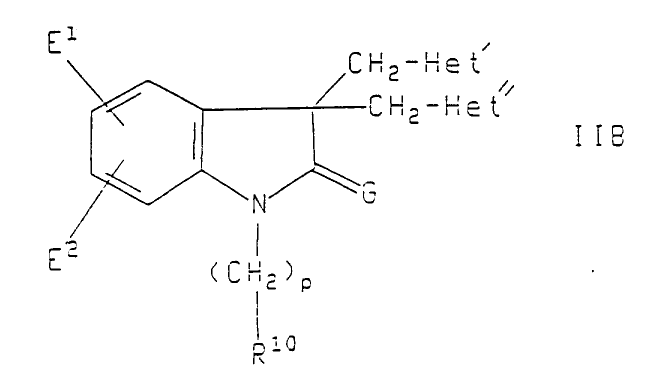 wherein A, X, G, Z, R³, R⁴, R⁵, R⁶, R⁷, R⁸, R¹⁰, E¹, E², p, Het' and Het" are defined as indicated in the specification. Novel compounds of the formula 1 are also claimed.

## Description

This invention relates to the use of certain substituted heterocyclic compounds for the treatment of cancer. The therapeutically active agents of this invention exhibit activity as inhibitors of the enzyme farnesyl protein transferase and are believed to be useful as anti-cancer and anti-tumor agents.

The therapeutic agents of this invention, i.e., compounds of the formulae I, IIA and IIB, as described below, and their pharmaceutically acceptable salts, as well as methods of preparing them, are referred to in International Patent Application PCT/US 92/11292, which designates the United States and was published on July 22, 1993 as WO 93/14085, United States Patent 4,876,259, which issued on October 24, 1989, International Patent Application PCT/IB 95/00189, which designates the United States and was filed on March 20, 1995, and United States Patent Application 08/236,743, which was filed on April 29, 1994. The foregoing patent and patent applications are incorporated herein by reference in their entireties.

Oncogenes frequently encode protein components of signal transduction pathways which lead to stimulation of cell growth and mitogenesis. Oncogene expression in cultured cells leads to cellular transformation, characterized by the ability of cells to grow in soft agar and the growth of cells as dense foci lacking the contact inhibition exhibited by non-transformed cells. Mutation and/or overexpression of certain oncogenes is frequently associated with human cancer.

To acquire transforming potential, the precursor of the Ras oncoprotein must undergo farnesylation of the cysteine residue located in a carboxyl-terminal tetrapeptide. Inhibitors of the enzyme that catalyzes this modification, farnesyl protein transferase, have therefore been suggested as anticancer agents for tumors in which Ras contributes to transformation. Mutated, oncogenic forms of Ras are frequently found in many human cancers, most notably in more than 50% of colon and pancreatic carcinomas (Kohl et al., Science, Vol. 260, 1834 to 1837, 1993).

### Summary of the Invention

This invention relates to a method of treating cancer in a mammal, including a human, comprising administering to said mammal an amount of a compound of the formula 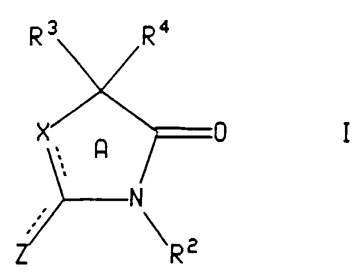
wherein both dotted lines represent optional double bonds;
Z is oxygen or sulfur when it is double bonded to ring A and Z is hydroxy, (C₁-C₁₀)alkyl-S-, (C₁-C₁₀)alkyl-SO-, (C₁-C₁₀)alkyl-SO₂-, adamant-2-yl-S-, naphthyl-S-, benzyl-S-, phenyl-C(=O)CH₂-S-, (C₁-C₆)alkyl-O-C(=O)-CH₂-S- or (H,H) (i.e., Z represents two hydrogen atoms, each of which is single bonded to the same carbon of ring A) when Z is single bonded to ring A, and wherein said naphthyl and phenyl and the phenyl moiety of said benzyl may optionally be substituted with from one to three substituents independently selected from (C₁-C₆)alkyl optionally substituted with from one to three fluorine atoms, (C₁-C₆)alkoxy optionally substituted with from one to three fluorine atoms, halo (e.g., chloro, fluoro, bromo or iodo), amino, (C₁-C₆)alkylamino, [di-(C₁-C₆)alkyl]amino, cyano, nitro, (C₁-C₆)alkyl-SOₙ- wherein n is zero, one or two, -COOH, -COO(C₁-C₆)alkyl and -C(O)NH(C₁-C₆)alkyl;
X is NR¹ or CHR¹;
R¹ is hydrogen, (C₁-C₆)alkyl or (C₁-C₆)alkylphenyl when ring A is saturated (i.e., when ring A contains no double bonds) and R¹ is absent when ring A contains a double bond;
R² is selected from naphthyl, phenyl, (C₁-C₆)alkylphenyl, 1-adamantyl, 2-adamantyl, (C₁-C₈) straight or branched alkyl, (C₃-C₁₀) cycloalkyl and (C₈-C₃₀)bicyclic or tricyclic alkyl; wherein said (C₃-C₁₀)cycloalkyl and said (C₈-C₃₀)bicyclic or tricyclic alkyl may optionally be substituted with a hydroxy group; and wherein said adamantyl groups may optionally be substituted with from one to three substituents independently selected from (C₁-C₆)alkyl, halo and hydroxy; and
R³ and R⁴ are independently selected from benzyl, wherein the phenyl moiety of said benzyl may optionally be substituted with an amino or nitro group; hydrogen, phenyl, (N ≡ C)-(C₁-C₆)alkyl, (C₁-C₆)alkyl-O-C(=O)-(C₁-C₆)alkyl and Het-CH₂, wherein Het is selected from 2-, 3- or 4-pyridinyl, furyl, tetrahydrofuryl, pyrimidyl, pyrazinyl, pyrazolyl, isoxazolyl, thiophenyl and triazolyl;
with the proviso that (a) no more than one of the two dotted lines can represent a double bond in any one compound, (b) when Z is (H, H), X is CH₂, (c) when Z is oxygen or (H, H) and X is CHR¹, R¹ must be hydrogen, (d) when Z is sulfur and X is NR¹, R¹ must be hydrogen, and (e) one of R³ and R⁴ must be Het-CH₂;
or a pharmaceutically acceptable salt thereof;
that is effective in treating cancer.

Preferred embodiments of this invention include the above methods wherein the compound of the formula I that is employed is a compound wherein one of R³ and R⁴ is 4-pyridinylmethyl.

Other preferred embodiments of this invention include the above methods wherein the compound of the formula I that is employed is a compound wherein Z = (benzyl)-S-, wherein the phenyl moiety of said benzyl may be optionally substituted as defined above, and R² is a cycloalkyl group.

This invention also relates to a method of treating cancer in a mammal, including a human, comprising administering to said mammal an amount of a compound of the formula 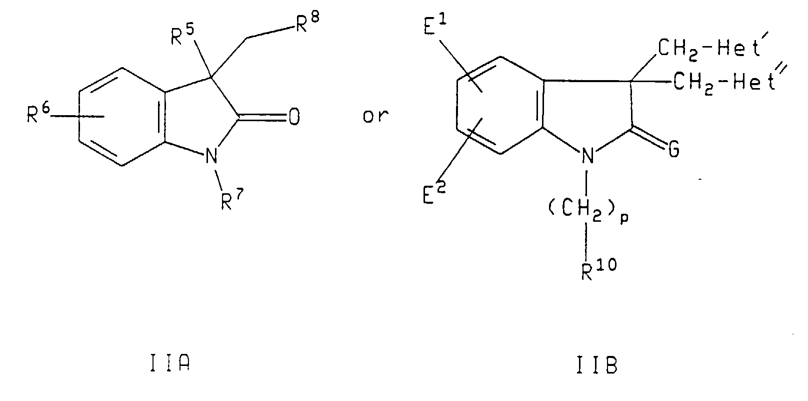 wherein R⁸ is 4-, 3-, or 2-pyridyl, pyrimidyl, pyrazinyl, 2-fluoro-4-pyridyl or 3-fluoro-4-pyridyl;
R⁷ is (C₁-C₁₀) straight or branched alkyl, (C₃-C₈)cycloalkyl, 2-, 3-, or 4-pyridyl, phenyl or phenyl substituted with W;
each W is, independently, chloro, fluoro, bromo, R⁹, -OH, -OR⁹, -NO₂, -NH₂, -NHR⁹, -NR⁹R⁹, -CN, or -S(O)ₘ-R⁹;
R⁶ is hydrogen, chloro, fluoro, bromo, -CN, -OH, -NO₂, -CF₃, -NHR⁹, -NR⁹R⁹, R⁹, -OR⁹ or -S(O)ₘ-R⁹;
each R⁹ is, independently, (C₁-C₄) straight or branched alkyl, phenyl or benzyl, wherein said phenyl and the phenyl moiety of said benzyl may optionally be substituted with chloro, fluoro, bromo, -CN, -OH, -NO₂, -CF₃, -NH₂, (C₁-C₄)alkylamino, di[(C₁-C₄)alkyl]amino or -S(O)ₘ-(C₁-C₄)alkyl;
R⁵ is -(CH₂)ₙ-Y or -OCOR⁹;
Y is hydrogen, OH, NH₂, -NHR⁹, -NR⁹R⁹, -NHCOR⁹, -NHCO₂R⁹, chloro, fluoro, bromo, OR⁹, -S(O)ₘR⁹, -CO₂H, -CO₂R⁹, -CN, -CONR⁹R⁹, -CONHR⁹, -CONH₂, -COR⁹; -CH=CHCO₂R⁹, -OCOR⁹, phenyl, phenyl substituted with W, -C ≡ CCO₂R⁹, -CH=CHR⁹ or -C ≡ CR⁹;
m is 0, 1 or 2;
n is 1 to 7;
p is 0 or 1;
G is oxygen or sulfur;
R¹⁰ is (C₁-C₁₀) straight or branched alkyl, (C₃-C₈) cycloalkyl, 2-, 3- or 4-pyridyl or 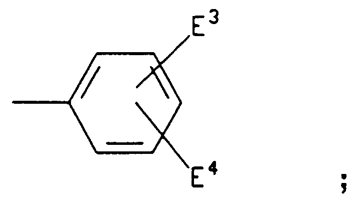
E¹, E², E³ and E⁴ are selected, independently, from hydrogen, halo (e.g., chloro, fluoro, bromo or iodo), (C₁-C₃)alkyl, -OH, (C₁-C₃)alkoxy, -NO₂, -CF₃, -CN, -NH₂, (C₁-C₃)alkylamino and di[(C₁-C₃)alkyl]amino;
Het' and Het" are selected, independently, from 6 membered heterocyclic rings containing at least one nitrogen atom as part of the ring, optionally substituted with one substituent selected from (C₁-C₃)alkyl, halo, -OH, (C₁-C₃)alkoxy, -NH₂, (C₁-C₃)alkylamino and di[(C₁-C₃)alkyl]amino;
or a pharmaceutically acceptable salt thereof,
that is effective in treating or preventing cancer.

More specific embodiments of this invention include methods of treating cancer, as described above, wherein the compounds administered are, respectively:
(a) a compound of the formula IIA;
(b) a compound of the formula IIB;
(c) a compound of the formula IIA wherein R⁶ is hydrogen;
(d) a compound of the formula IIA wherein R⁵ is -(CH₂)ₙ-Y;
(e) a compound of the formula IIA wherein R⁵ is -OCOR⁹;
(f) a compound of the formula IIA wherein R⁸ is 4-, 3- or 2-pyridyl;
(g) a compound of the formula IIA wherein R⁷ is 4-, 3- or 2-pyridyl;
(h) a compound of the formula IIB wherein E¹ and E² are both hydrogen;
(i) a compound of the formula IIB wherein R¹⁰ is 2-, 3- or 4-pyridyl;
(j) a compound of the formula IIB wherein one or both of Het' and Het" is 2-, 3- or 4-pyridyl;
(k) a compound of the formula IIB wherein one or both of Het' and Het" is pyrimidyl; and
(I) a compound of the formula IIB wherein each of Het' and Het" contains from one to three ring nitrogen atoms.

This invention also relates to a method of inhibiting the abnormal growth of cells in a mammal, including a human, comprising administering to said mammal a farnesyl protein transferase inhibiting effective amount of a compound of the formula I, IIA or IIB, as defined above, or a pharmaceutically acceptable salt of such a compound.

This invention also relates to a method of inhibiting the abnormal growth of cells in a mammal, including a human, comprising administering to said mammal an abnormal cell growth inhibiting effective amount of a compound of the formula I, IIA or IIB, as defined above, or a pharmaceutically acceptable salt of such a compound.

"Abnormal cell growth", as used herein, refers to cell growth that is independent of normal regulatory mechanisms (e.g., loss of contact inhibition). This includes the abnormal growth of: (1) tumor cells (tumors) expressing an activated Ras oncogene; (2) tumor cells in which the Ras protein is activated as a result of oncogenic mutation in another gene; and (3) benign and malignant cells of other proliferative diseases in which aberrant Ras activation occurs.

Examples of such benign proliferative diseases are psoriasis, benign prostatic hypertrophy and restinosis.

The term "alkyl", as used herein, unless otherwise indicated, includes saturated monovalent hydrocarbon radicals having straight, branched or cyclic moieties or combinations thereof.

The term "halo", as used herein, refers to chloro, fluoro, bromo or iodo.

The compounds of formulae I, IIA and IIB that are basic in nature are capable of forming a wide variety of salts with various inorganic and organic acids. The acids that may be used to prepare pharmaceutically acceptable acid addition salts of those compounds of formulae I, IIA and IIB that are basic in nature are those that form non-toxic acid addition salts, i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate and pamoate [i.e., 1,1'-methylene-bis-(2-hydroxy-3-naphthoate)] salts.

The compounds of formulae I, IIA and IIB above may contain chiral centers and therefore may exist in different enantiomeric forms. This invention relates to any of the above methods that employ any of the optical isomers or other stereoisomers of compounds of the formulae I, IIA and llB and mixtures thereof.

Patients that can be treated with compounds of the formula I, IIA or IIB according to the methods of this invention include, for example, patients that have been diagnosed as having lung cancer, bone cancer, pancreatic cancer, skin cancer, cancer of the head and neck, cutaneous or intraocular melanoma, uterine cancer, ovarian cancer, rectal cancer, cancer of the anal region, stomach cancer, colon cancer, breast cancer, gynecologic tumors (e.g., uterine sarcomas, carcinoma of the fallopian tubes, carcinoma of the endometrium, carcinoma of the cervix, carcinoma of the vagina or carcinoma of the vulva), Hodgkin's Disease, cancer of the esophagus, cancer of the small intestine, cancer of the endocrine system (e.g., cancer of the thyroid, parathyroid or adrenal glands), sarcomas of soft tissues, cancer of the urethra, cancer of the penis, prostate cancer, chronic or acute leukemia, solid tumors of childhood, lymphocytic lymphonas, cancer of the bladder, cancer of the kidney or ureter (e.g., renal cell carcinoma, carcinoma of the renal pelvis), or neoplasms of the central nervous system (e.g., primary CNS lymphona, spinal axis tumors, brain stem gliomas or pituitary adenomas).

Patients that can be treated with compounds of the formula I, IIA or IIB according to the methods of this invention also include patients suffering from abnormal cell growth, as defined above.

### Detailed Description Of The Invention

The preparation of compounds of the formulae I, IIA and IIB are described below. In the reaction schemes and discussion that follow, X, Y, Z, W, A, R¹, R², R³, R⁴, R⁵, R⁶, R⁷, R⁸, R⁹, R¹⁰, E¹, E², E³, E⁴, G, Het, Het' and Het" are defined as above.

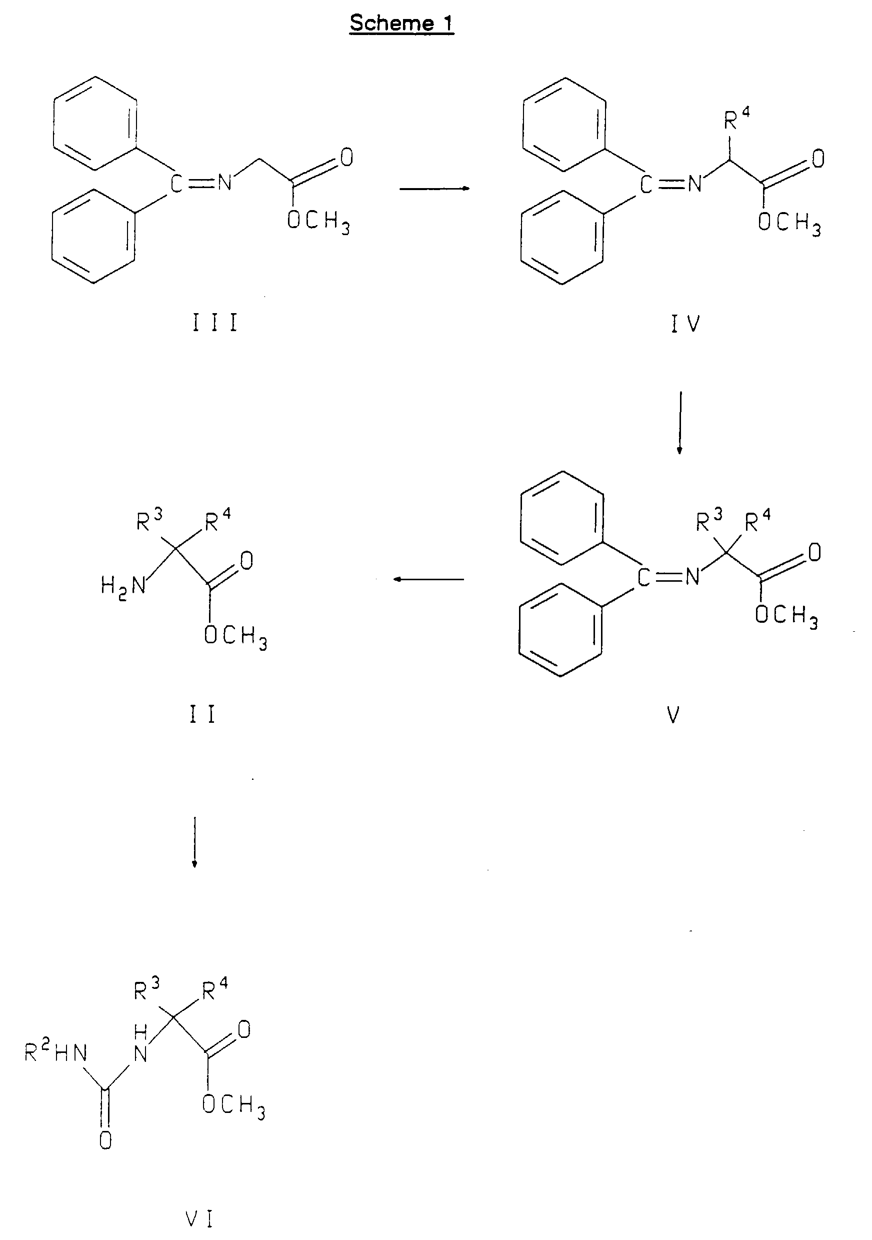

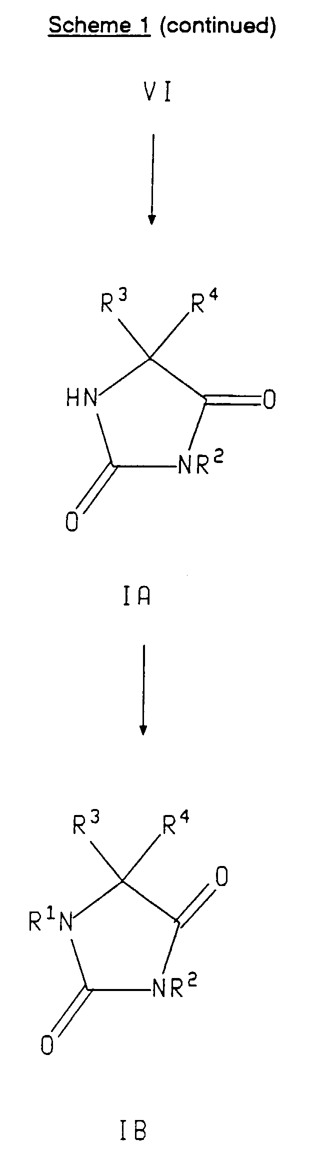

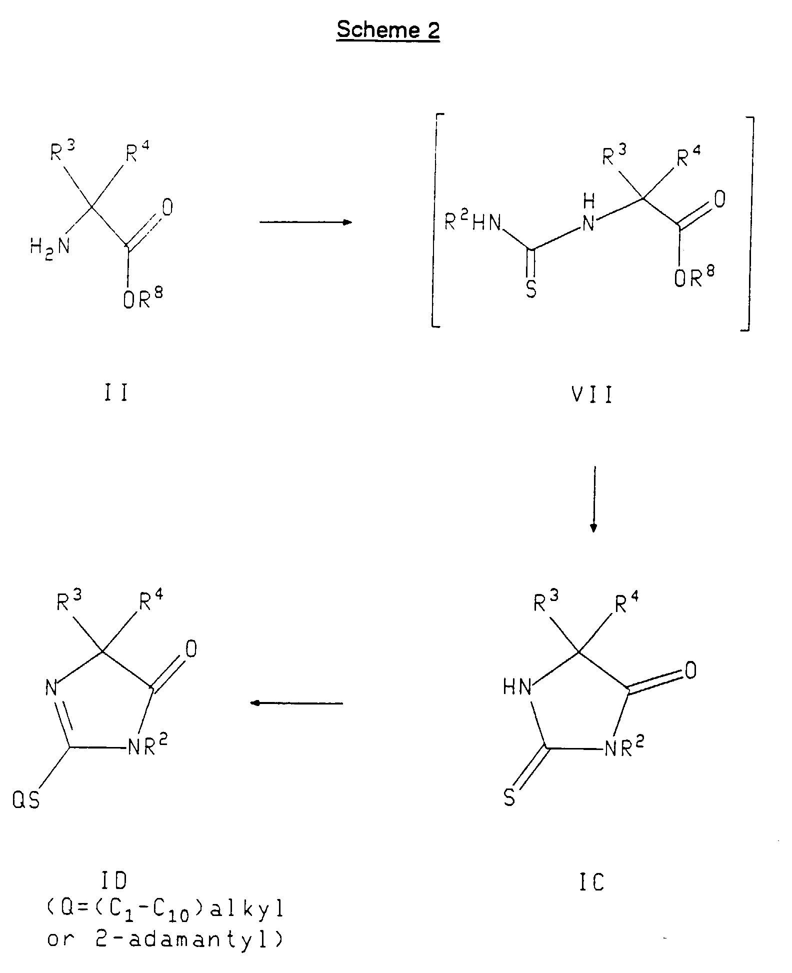

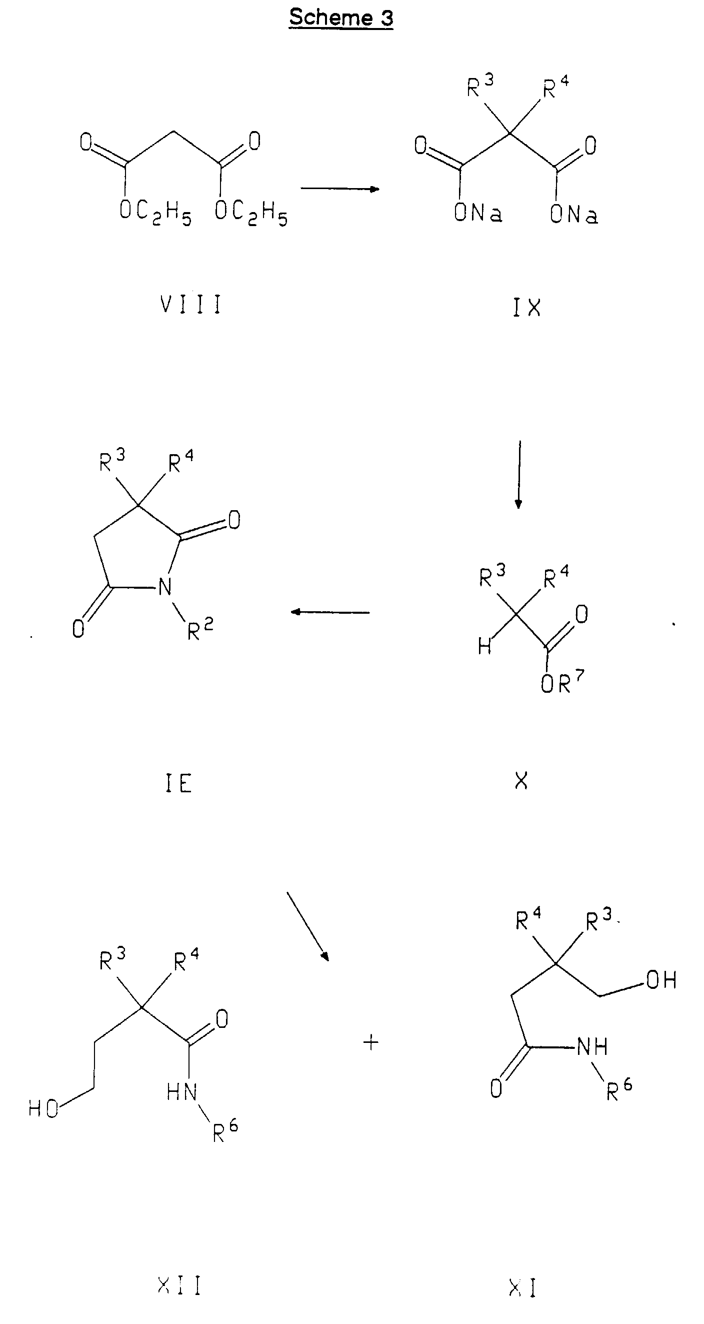

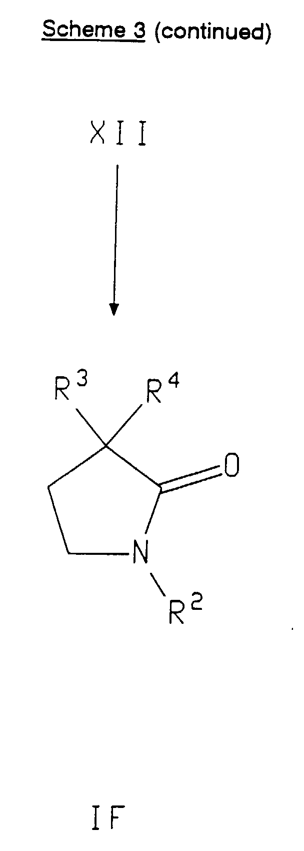

Referring to Scheme 1, a compound of the formula III is reacted with potassium bis(trimethylsilyl)amide in THF (tetrahydrofuran) at a temperature of about -70°C. After stirring for about 30 minutes, a compound of the formula 2-, 3- or 4-pyridinylmethyl-X, wherein X is an appropriate leaving group (e.g., chloride or bromide), is added and the reaction mixture is allowed to warm to about ambient temperature. This reaction yields a compound of the formula IV wherein R⁴ is 2-, 3- or 4-pyridinylmethyl, which can be isolated or reacted in situ to form a compound of the formula V.

Addition of the R³ substituent to the compound of formula IV yields a compound of the formula V. This is accomplished by carrying out the same procedure described above for making the compound of formula IV, with the exception that a compound of the formula R³X is used instead of 2-, 3- or 4-pyridinylmethyl-X. (R³X may, however, as indicated above, be 2, 3-or 4-pyridinylmethyl-X.)

Compounds of the formula II may be formed by reacting the corresponding compounds of the formula V with an acid. The acid is preferably a mineral acid such as hydrochloric, nitric or sulfuric acid. This reaction is typically carried out using an organic cosolvent such as ethyl ether, tetrahydrofuran (THF) or acetonitrile, preferably THF. The reaction temperature may range from about -5°C to about 35°C, and is preferably between about 0°C and about room temperature.

Reaction of the resulting compound of formula II with an isocyanate of the formula R²NCO yields the corresponding urea of formula VI. Generally, this reaction is carried out in a protic solvent such as methanol, ethanol or methylene chloride, with methanol and ethanol being preferred, at a temperature from about room temperature to about 78°C, preferably at about the reflux temperature of the solvent. The reaction is preferably carried out for about six to eight hours, but can be carried out for longer or shorter periods (e.g., from about a half day to about two days).

The urea of formula VI can be isolated or converted in situ to the corresponding hydantoin derivative of formula IA. (Thin layer chromatography (TLC) can be used to determine when the starting material from the preceding reaction has been consumed.) The conversion is effected by heating the compound of formula VI in the presence of a catalytic amount of potassium cyanide in a reaction inert solvent that is the same or similar to that used in the preceding reaction. This reaction is preferably conducted at the reflux temperature of the solvent, though lower temperatures (e.g., about ambient temperature to about 78°C) are also suitable.

Compounds of the formula IB may be formed by reacting the corresponding compounds of the formula IA with a strong base (e.g., sodium hydride, lithium diisopropylamide, lithium hydride or potassium hydride), followed by a compound of the formula R¹X, wherein X is a leaving group (e.g., chloride , iodide or bromide), and the preferred base is sodium hydride and the preferred leaving group is iodide or bromide. The solvent is typically an aprotic solvent such as THF, dimethylformamide (DMF) or an ether such as ethyl ether. It is preferably DMF. The reaction temperature can range from about -78°C to about 70°C. A temperature of about 0°C to about room temperature is preferred.

Referring to Scheme 2, compounds of the formula IC and ID may be formed as follows. A compound of the formula II is reacted with a compound of the formula R²NCS to form the corresponding compound of formula IC. This reaction, which proceeds through an intermediate of the formula VII, is generally carried out using similar solvents and under similar conditions as those described above for the formation of the urea of formula VI. When R² is adamantyl, it is preferable to use a large excess of the reactant R²NCS and to let the reaction proceed for a period of about two days to one week.

The resulting compound of formula IC can then be converted into the corresponding compound of formula ID by reacting it with a compound of the formula QX wherein Q is (C₁-C₆)alkyl, 2-adamantyl, phenyl-C(=O)CH₂- or (C₁-C₆)alkyl-O-C(=O)-CH₂- and X is a leaving group, as defined above. This reaction is typically carried out in a polar solvent such as THF, DMF or acetonitrile or acetone, preferably acetone, in the presence of a base scavenger such as a carbonate or an organic tertiary amine. Potassium carbonate is preferred. The reaction temperature can range from about -78°C to about 140°C, with about 0°C to about room temperature being preferred. When Q is adamantyl or another bulky substituent such as cyclohexyl, the reaction is preferably carried out in DMF at a temperature from about 25°C to about the reflux temperature.

Referring to Scheme 3, compounds of the formula IX may be prepared by reacting diethylmalonate with sodium ethoxide, followed by 2-, 3- or 4-pyridinylmethyl-X, wherein X is a leaving group (e.g., chloro or bromo). The monoalkylated product is then reacted with sodium ethoxide, followed by a compound of the formula R³X, wherein X is a leaving group, as defined above. (The reactions with 2-, 3- or 4-pyridylmethyl-X and R³X can be carried out simultaneously or sequentially. However, when R³ and R⁴ are different, it is preferable to isolate and purify the monoalkylated product prior to formation of the dialkylated product with R³X.) The bisester is then hydrolyzed with two to three equivalents of sodium or potassium hydroxide. Preferably, after addition of the sodium or potassium hydroxide, the reaction mixture is stirred for up to about 48 hours. These ester hydrolysis reactions are generally conducted at a temperature of about 0°C to about 60°C in an ether/alcohol/water solvent, preferably a THF:methanol:water mixture.

The compound of formula IX can be converted into the corresponding compound of the formula X by reacting it at a temperature of about 0°C to about 50°C with an excess of an anhydrous acid such as hydrochloric or hydrobromic acid, in the amount of one equivalent of acid per basic functionality in the compound of formula IX (e.g., four equivalents of acid if both R³ and R³ are 4-pyridinylmethyl), and with an alcohol of the formula R⁷OH, and then evaporating the reaction mixture to dryness.

Alternatively, when R³ and R⁴ are acid sensitive, compounds of the formula X can be prepared as described by Krapcho et al., J. Org. Chem., 43, 138 (1978). This procedure involves treatment of the bis-alkylated malonate with dimethylsulfoxide (DMSO), lithium chloride and water.

The pyrrolidine-2,5-dione derivative of formula IE may be prepared by reacting the corresponding compound of formula X with an amide base followed by a compound of the formula R²NHCOCH₂X, wherein X is a leaving group, as defined above. Appropriate bases include lithium diisopropylamide, lithium hexamethyldisilazide and lithium diethylamide. Suitable solvents include aprotic solvents such as THF, ethyl ether, DMF, benzene and toluene. The reaction may be conducted at temperatures ranging from about -78°C to about room temperature. Preferably, this reaction is carried out in THF, using lithium diisopropylamide as the base and bromide as the leaving group, at a temperature from about -78°C to about room temperature.

Reaction of the resulting compound of the formula IE with a borohydride yields the corresponding compounds of the formulae XII and XI. The major product formed is the compound of formula XII. This reaction is generally carried out in a protic solvent such as a lower alcohol, preferably isopropanol, at a temperature from about -20°C to about 50°C, preferably at about room temperature, using a large excess of borohydride. Sodium borohydride is the preferred reactant, though other borohydrides (e.g., lithium borohydride) may also be used.

The compounds of formula XII formed in the above step can be converted into the corresponding compounds of the formula IF by reacting them with a phosphine (e.g., tributylphosphine or triphenylphosphine) and an azodicarboxylate (e.g., diisopropylazodicarboxylate or diethylazodicarboxylate). Suitable solvents for this reaction (a Mitsunobu reaction) include aprotic solvents such as THF, methylene chloride or acetonitrile, with THF being preferred. Suitable temperatures range from about 0°C to about 40°C, with about room temperature being preferred.

World Patent Application WO 93/14085, referred to above and incorporated herein by reference in its entirety, describes a method of preparing compounds of the formula IIA.

United States Patent Application 4,876,259, also referred to above and incorporated herein by reference in its entirety, describes methods of synthesizing compounds of the formula llB.

The compounds of the formulae I, IIA and llB that are basic in nature are capable of forming a wide variety of different salts with various inorganic and organic acids. Although such salts must be pharmaceutically acceptable for administration to animals, it is often desirable in practice to initially isolate the compound of formula I, llA and IIB from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained. The desired acid salt can also be precipitated from a solution of the free base in an organic solvent by adding to the solution an appropriate mineral or organic acid.

The compounds of the formulae I, IIA and IIB exhibit activity as Ras farnesylation inhibitors and are useful in the treatment of cancer and the inhibition of abnormal cell growth in mammals, including humans.

Patients that can be treated with compounds of the formula I, IIA or IIB according to the methods of this invention include, for example, patients that have been diagnosed as having lung cancer, bone cancer, pancreatic cancer, skin cancer, cutaneous or intraocular melanoma, uterine cancer, ovarian cancer, rectal cancer, cancer of the anal region, stomach cancer, colon cancer, breast cancer, gynecologic tumors (e.g., uterine sarcomas, carcinoma of the fallopian tubes, carcinoma of the endometrium, carcinoma of the cervix, carcinoma of the vagina or carcinoma of the vulva), Hodgkin's Disease, cancer of the esophagus, cancer of the small intestine, cancer of the endocrine system (e.g., cancer of the thyroid, parathyroid or adrenal glands), sarcomas of soft tissues, cancer of the urethra, cancer of the penis, prostrate cancer, chronic or acute leukemia, solid tumors of childhood, lymphocytic lymphonas, cancer of the bladder, cancer of the kidney or ureter (e.g., renal cell carcinoma, carcinoma of the renal pelvis), or neoplasms of the central nervous system (e.g., primary CNS lymphona, spinal axis tumors, brain stem gliomas or pituitary adenomas).

The compounds of formulae I, IIA and llB and their pharmaceutically acceptable salts (hereinafter referred to, collectively, as "the therapeutic compounds") can be administered orally, transdermally (e.g., through the use of a patch), parenterally or topically. Oral administration is preferred. In general, compounds of the formula I and their pharmaceutically acceptable salts are most desirably administered in dosages ranging from about 1.0 mg up to about 500 mg per day, preferably from about 1 to about 100 mg per day in single or divided (i.e., multiple) doses. Compounds of the formulae IIA and llB and their pharmaceutically acceptable salts will ordinarily be administered in daily dosages ranging from about 0.01 to about 10 mg per kg body weight per day, in single or divided doses. Variations may occur depending on the weight and condition of the person being treated and the particular route of administration chosen. In some instances, dosage levels below the lower limit of the aforesaid range may be more than adequate, while in other cases still larger doses may be employed without causing any harmful side effect, provided that such larger doses are first divided into several small doses for administration throughout the day.

The therapeutic compounds may be administered alone or in combination with pharmaceutically acceptable carriers or diluents by either of the two routes previously indicated, and such administration may be carried out in single or multiple doses. More particularly, the novel therapeutic compounds of this invention can be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, elixirs, syrups, and the like. Such carriers include solid diluents or fillers, sterile aqueous media and various non-toxic organic solvents, etc. Moreover, oral pharmaceutical compositions can be suitably sweetened and/or flavored.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic compound in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intra-articular, intra-muscular and subcutaneous injection purposes. The preparation of all these solutions under sterile conditions is readily accomplished by standard pharmaceutical techniques well-known to those skilled in the art.

Additionally, it is also possible to administer the therapeutic compounds topically and this may preferably be done by way of creams, jellies, gels, pastes, ointments and the like, in accordance with standard pharmaceutical practice.

The activity of the therapeutic compounds as ras farnesylation inhibitors may be determined by their ability, relative to a control, to inhibit ras farnesyl transferase in vitro. This procedure is described below.

A crude preparation of human farnesyl transferase (FTase) comprising the cytosolic fraction of homogenized brain tissue is used for screening compounds in a 96-well assay format. The cytosolic fraction is prepared by homogenizing approx. 40 grams fresh tissue in 100 ml of sucrose/MgCl₂/EDTA buffer (using a Dounce homogenizer; 10-15 strokes), centrifuging the homogenates at 1000 grams for 10 minutes at 4G, re-centrifuging the supematant at 17,000 grams for 15 minutes at 4G, and then collecting the resulting supernatant. This supernatant is diluted to contain a final concentration of 50 mM Tris HCI (pH 7.5), 5 mN DTT, 0.2 M KCI, 20 µM ZnCl₂, 1 mM PMSF and re-centrifuged at 178,000 grams for 90 minutes at 4G. The supernatant, termed "crude FTase" was assayed for protein concentration, aliquoted, and stored at -70°C.

The assay used to measure in vitro inhibition of human FTase is a modification of the method described by Amersham LifeScience for using their Farnesyl transferase (3H) Scintilation Proximity Assay (SPA) kit (TRKQ 7010). FTase enzyme activity is determined in a volume of 100 µl containing 50 mM N-(2-hydroxy ethyl) piperazine-N'-(2-ethane sulfonic acid) (HEPES), pH 7.5, 30 mM MgCl₂, 20 uM KCI, 5 mM Na₂HPO₄, 5 mM dithiothreitol (DTT), 0.01% Triton X-100, 5% dimethyl sulfoxide (DMSO), 20 µg of crude FTase, 0.12 µM [3H]-farnesyl pyrophosphate ([3H]-FPP; 36000 dpm/pmole, Amersham LifeScience), and 0.2 µM of biotinylated Ras peptide KTKCVIS (Bt-KTKCVIS) that is N-terminally biotinylated at its alpha amino group and was synthesized and purified by HPLC in house. The reaction is initiated by addition of the enzyme and terminated by addition of EDTA (supplied as the STOP reagent in kit TRKQ 7010) following a 45 minute incubation at 37°C. Prenylated and unprenylated Bt-KTKCVIS is captured by adding 10 µl of steptavidin-coated SPA beads (TRKQ 7010) per well and incubating the reaction mixture for 30 minutes at room temperature. The amount of radioactivity bound to the SPA beads is determined using a MicroBeta 1450 plate counter. Under these assay conditions, the enzyme activity is linear with respect to the concentrations of the prenyl group acceptor, Bt-KTKCVIS, and crude FTase, but saturating with respect to the prenyl donor, FPP. The assay reaction time is also in the linear range.

The test compounds are routinely dissolved in 100% DMSO. Inhibition of farnesyl transferase activity is determined by calculating percent incorporation of tritiated-farnesyl in the presence of the test compound vs. its incorporation in control wells (absence of inhibitor). IC₅₀ values, that is, the concentration required to produce half maximal farnesylation of Bt-KTKCVIS, is determined from the dose-responses obtained.

## Claims

1. A compound of the formula 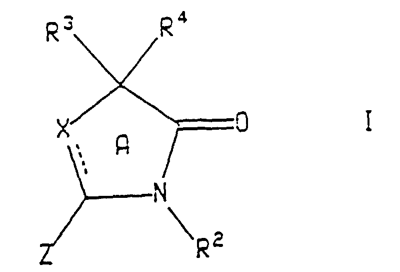
wherein both dotted lines represent optional double bonds;
Z is (C₁-C₁₀)alkyl-SO-, (C₁-C₁₀)alkyl-SO₂-, naphthyl-S-, benzyl-S-, or phenyl-C(=O)CH₂-S-, wherein the naphthyl moiety of said naphthyl-S- may optionally be substituted and said phenyl and the phenyl moiety of said benzyl are substituted with from one to three substituents independently selected from (C₁-C₆)alkyl optionally substituted with from one to three fluorine atoms, (C₁-C₆)alkoxy optionally substituted with from one to three fluorine atoms, halo, amino, (C₁-C₆)alkylamino, [di-(C₁-C₆)alkyl]amino, cyano, nitro, (C₁-C₆)alkyl-SOₙ- wherein n is zero; one or two, -COOH, -COO(C₁-C₆)alkyl and -C(O)NH(C₁-C₆)alkyl;
X is NR¹ or CHR¹;
R¹ is hydrogen, (C₁-C₆)alkyl or (C₁-C₆)alkylphenyl when ring A is saturated and R¹ is absent when ring A contains a double bond;
R² is selected from naphthyl, phenyl, (C₁-C₆)alkylphenyl, 1-adamantyl, 2-adamantyl, (C₁-C₈) straight or branched alkyl, (C₃-C₁₀) cycloalkyl and (C₈-C₃₀)bicyclic or tricyclic alkyl; wherein said (C₃-C₁₀)cycloalkyl and said (C₈-C₃₀)bicyclic or tricyclic alkyl may optionally be substituted with a hydroxy group; and wherein said adamantyl groups may optionally be substituted with from one to three substituents independently selected from (C₁-C₆)alkyl, halo and hydroxy; and
R³ and R⁴ are independently selected from benzyl, wherein the phenyl moiety of said benzyl may optionally be substituted with an amino or nitro group; hydrogen, phenyl, (N ≡ C)-(C₁-C₆)alkyl, (C₁-C₆)alkyl-O-C(=O)-(C₁-C₆)alkyl and Het-CH₂, wherein Het is selected from 2-, 3- or 4-pyridinyl, furyl, tetrahydrofuryl, pyrimidyl, pyrazinyl, pyrazolyl, isoxazolyl, thiophenyl and triazolyl;
with the proviso that one of R³ and R⁴ must be Het-CH₂;
or a pharmaceutically acceptable salt thereof.

2. The use of a compound of the formula 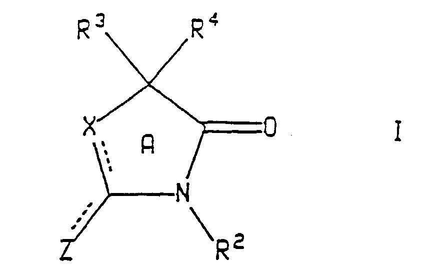
wherein both dotted lines represent optional double bonds;
Z is oxygen or sulfur when it is double bonded to ring A and Z is hydroxy, (C₁-C₁₀)alkyl-S-, (C₁-C₁₀)alkyl-SO-, (C₁-C₁₀)alkyl-SO₂-, adamant-2-yl-S-, naphthyl-S-, benzyl-S-, phenyl-C(=O)CH₂-S-, (C₁-C₆)alkyl-O-C(=O)-CH₂-S- or (H,H) when Z is single bonded to ring A, and wherein said naphthyl and phenyl and the phenyl moiety of said benzyl may optionally be substituted with from one to three substituents independently selected from (C₁-C₆)alkyl optionally substituted with from one to three fluorine atoms, (C₁-C₆)alkoxy optionally substituted with from one to three fluorine atoms, halo, amino, (C₁-C₆)alkylamino, [di-(C₁-C₆)alkyl]amino, cyano, nitro, (C₁-C₆)alkyl-SOₙ- wherein n is zero, one or two, -COOH, -COO(C₁-C₆)alkyl and -C(O)NH(C₁-C₆)alkyl;
X is NR¹ or CHR¹;
R¹ is hydrogen, (C₁-C₆)alkyl or (C₁-C₆)alkylphenyl when ring A is saturated and R¹ is absent when ring A contains a double bond;
R² is selected from naphthyl, phenyl, (C₁-C₆)alkylphenyl, 1-adamantyl, 2-adamantyl, (C₁-C₈) straight or branched alkyl, (C₃-C₁₀) cycloalkyl and (C₃-C₃₀)bicyclic or tricyclic alkyl; wherein said (C₃-C₁₀)cycloalkyl and said (C₃-C₃₀)bicyclic or tricyclic alkyl may optionally be substituted with a hydroxy group; and wherein said adamantyl groups may optionally be substituted with from one to three substituents independently selected from (C₁-C₆)alkyl, halo and hydroxy; and
R³ and R⁴ are independently selected from benzyl, wherein the phenyl moiety of said benzyl may optionally be substituted with an amino or nitro group; hydrogen, phenyl, (N ≡ C)-(C₁-C₆)alkyl, (C₁-C₆)alkyl-O-C(=O)-(C₁-C₆)alkyl and Het-CH₂, wherein Het is selected from 2-, 3- or 4-pyridinyl, furyl, tetrahydrofuryl, pyrimidyl, pyrazinyl, pyrazolyl, isoxazolyl, thiophenyl and triazolyl;
with the proviso that (a) no more than one of the two dotted lines can represent a double bond in any one compound, (b) when Z is (H, H), X is CH₂, (c) when Z is oxygen or (H, H) and X is CHR¹, R¹ must be hydrogen, (d) when Z is sulfur and X is NR¹, R¹ must be hydrogen, and (e) one of R³ and R⁴ must be Het-CH₂;
or a pharmaceutically acceptable saft thereof:
for the manufacture of a medicament for the treatment or prevention of cancer in a mammal.

3. The use according to claim 2, wherein one of R³ and R⁴ is 4-pyridinylmethyl.

4. The use according to claim 2 or 3, wherein X is benzyl-S-, wherein the phenyl moiety of the benzyl group may be optionally substituted as defined in claim 2.

5. The use of a compound of the formula 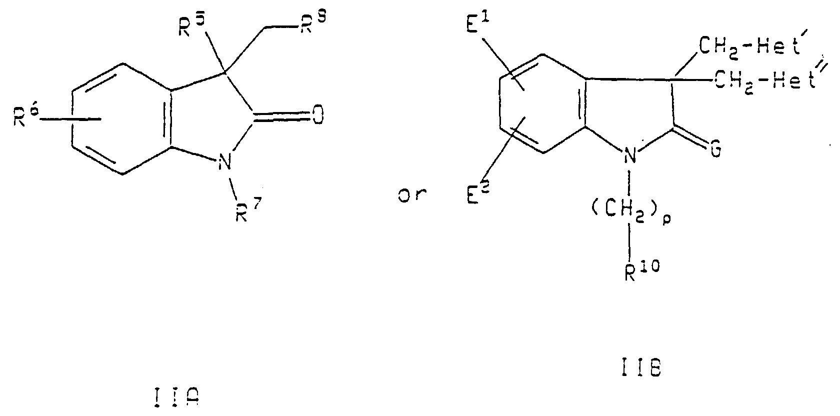
wherein R⁸ is 4-, 3-, or 2-pyridyl, pyrimidyl, pyrazinyl, 2-fluoro-4-pyridyl or 3-fluoro-4-pyridyl;
R⁷ is (C₁-C₁₀) straight or branched alkyl, (C₃-C₈)cycloalkyl, 2-, 3-, or 4-pyridyl, phenyl or phenyl substituted with W;
each W is, independently, fluoro, chloro, bromo, R⁹, -OH, -OR⁹, -NO₂, -NH₂, -NHR⁹, -NR⁹R⁹, -CN, or -S(O)ₘ-R⁹;
R⁶ is hydrogen, fluoro, chloro, bromo, Br, -CN, -OH, -NO₂, -CF₃, -NHR⁹, -NR⁹R⁹, R⁹, -OR⁹ or -S(O)ₘ-R⁹;
each R⁹ is, independently, (C₁-C₄) straight or branched alkyl, phenyl or benzyl, wherein said phenyl and the phenyl moiety of said benzyl may optionally be substituted with chloro, fluoro, bromo, -CN, -OH, -NO₂, -CF₃, -NH₂, (C₁-C₄)alkylamino, di[(C₁-C₄)alkyl]amino or -S(O)ₘ-(C₁-C₄)alkyl;
R⁵ is (CH₂)ₙ-Y or -OCOR⁹;
Y is hydrogen, OH, NH₂, -NH₂, -NHR⁹, -NR⁹R⁹, -NHCOR⁹, -NHCO₂R⁹, fluoro, chloro, bromo, OR⁹, -S(O)ₘR⁹, -CO₂H, -CO₂R⁹, -CN, -CONR⁹R⁹, -CONHR⁹, -CONH₂, -COR⁹; -CH=CHCO₂R⁹, -OCOR⁹, phenyl, phenyl substituted with W, -C≡CCO₂R⁹, -CH=CHR⁹ or -C≡CR⁹;
m is 0, 1 or 2;
n is 1 to 7;
p is 0 or 1;
G is oxygen or sulfur;
R¹⁰ is (C₁-C₁₀) straight or branched alkyl, (C₃-C₈) cycloalkyl, 2-, 3- or 4-pyridyl or 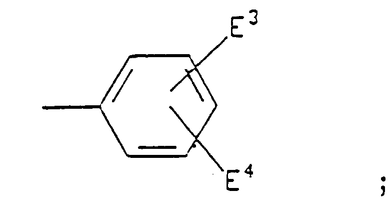 E¹, E², E³ and E⁴ are selected, independently, from hydrogen, halo, (C₁-C₃)alkyl, -OH, (C₁-C₃)alkoxy, -NO₂, -CF₃, -CN, -NH₂, (C₁-C₃)alkylamino and di[(C₁-C₃)alkyl]amino;
Het' and Het" are selected, independently, from 6 membered heterocyclic rings containing at least one nitrogen atom as part of the ring, optionally substituted with one substituent selected from (C₁-C₃)alkyl, halo, -OH, (C₁-C₃)alkoxy, -NH₂, (C₁-C₃)alkylamino and di[(C₁-C₃)alkyl]amino;
or a pharmaceutically acceptable salt thereof; for the manufacture of a medicament for the treatment or prevention of cancer in a mammal.

6. The use according to any one of claims 2 to 5 for treating breast cancer, bone cancer, lung cancer, pancreatic cancer, skin cancer, prostate cancer or colon cancer.

7. The use of a compound of the formula 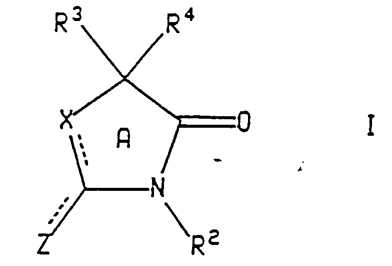
wherein both dotted lines represent optional double bonds;
Z is oxygen or sulfur when it is double bonded to ring A and Z is hydroxy, (C₁-C₁₀)alkyl-S-, (C₁-C₁₀)alkyl-SO-, (C₁-C₁₀)alkyl-SO₂-, adamant-2-yl-S-, naphthyl-S-, benzyl-S-, phenyl-C(=O)CH₂-S-, (C₁-C₆)alkyl-O-C(=O)-CH₂-S- or (H,H) when Z is single bonded to ring A, and wherein said naphthyl and phenyl and the phenyl moiety of said benzyl may optionally be substituted with from one to three substituents independently selected from (C₁-C₆)alkyl optionally substituted with from one to three fluorine atoms, (C₁-C₆)alkoxy optionally substituted with from one to three fluorine atoms, halo, amino, (C₁-C₆)alkylamino, [di-(C₁-C₆)alkyl]amino, cyano, nitro, (C₁-C₆)alkyl-SOₙ- wherein n is zero, one or two, -COOH, -COO(C₁-C₆)alkyl and -C(O)NH(C₁-C₆)alkyl;
X is NR¹ or CHR¹;
R¹ is hydrogen, (C₁-C₆)alkyl or (C₁-C₆)alkylphenyl when ring A is saturated and R¹ is absent when ring A contains a double bond;
R² is selected from naphthyl, phenyl, (C₁-C₆)alkylphenyl, 1-adamantyl, 2-adamantyl, (C₁-C₈) straight or branched alkyl, (C₃-C₁₀) cycloalkyl and (C₈-C₃₀)bicyclic or tricyclic alkyl; wherein said (C₈-C₁₀)cycloalkyl and said (C₃-C₃₀)bicyclic or tricyclic alkyl may optionally be substituted with a hydroxy group; and wherein said adamantyl groups may optionally be substituted with from one to three substituents independently selected from (C₁-C₆)alkyl, halo and hydroxy; and
R³ and R⁴ are independently selected from benzyl, wherein the phenyl moiety of said benzyl may optionally be substituted with an amino or nitro group; hydrogen, phenyl, (N ≡ C)-(C₁-C₆)alkyl, (C₁-C₆)alkyl-O-C(=O)-(C₁-C₆)alkyl and Het-CH₂, wherein Het is selected from 2-, 3- or 4-pyridinyl, furyl, tetrahydrofuryl, pyrimidyl, pyrazinyl, pyrazolyl, isoxazolyl, thiophenyl and triazolyl;
with the proviso that (a) no more than one of the two dotted lines can represent a double bond in any one compound, (b) when Z is (H, H), X is CH₂, (c) when Z is oxygen or (H, H) and X is CHR¹, R¹ must be hydrogen, (d) when Z is sulfur and X is NR¹, R¹ must be hydrogen, and (e) one of R³ and R⁴ must be Het-CH₂:
or a pharmaceutically acceptable salt thereof;for the manufacture of a medicament for the inhibition of abnormal cell growth in a mammal.

8. The use of a compound of formula 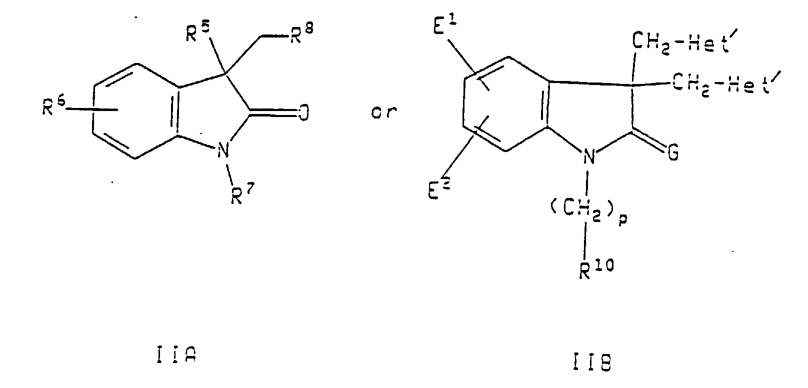
wherein R⁸ is 4-, 3-, or 2-pyridyl, pyrimidyl, pyrazinyl, 2-fluoro-4-pyridyl or 3-fluoro-4-pyridyl;
R⁷ is (C₁-C₁₀) straight or branched alkyl, (C₃-C₈)cycloalkyl, 2-, 3-, or 4-pyridyl, phenyl or phenyl substituted with W;
each W is, independently, fluoro, chloro, bromo, R⁹, -OH, -OR⁹, -NO₂, -NH₂, -NHR⁹, -NR⁹R⁹, -CN, or -S(O)ₘ-R⁹;
R⁶ is hydrogen, fluoro, chloro, bromo, -CN, -OH, -NO₂, -CF₃, -NHR⁹, -NR⁹R⁹, R⁹, -OR⁹ or -S(O)ₘ-R⁹;
each R⁹ is, independently, (C₁-C₄) straight or branched alkyl, phenyl or benzyl, wherein said phenyl and the phenyl moiety of said benzyl may optionally be substiuted with chloro, fluoro, bromo, -CN, -OH, -NO₂, -CF₃, -NH₂, (C₁-C₄)alkylamino, di[(C₁-C₄)alkyl]amino or -S(O)ₘ-(C₁-C₄)alkyl;
R⁵ is -(CH₂)ₙ-Y or -OCOR⁹;
Y is hydrogen, OH, NH₂, -NH₂, -NHR⁹, -NR⁹R⁹, -NHCOR⁹, -NHCO₂R⁹, fluoro, chloro, bromo, OR⁹, -S(O)ₘR⁹, -CO₂H, -CO₂R⁹, -CN, -CONR⁹R⁹, -CONHR⁹, -CONH₂, -COR⁹; -CH=CHCO₂R⁹, -OCOR⁹, phenyl, phenyl substituted with W, -C≡CCO₂R⁹, -CH=CHR⁹ or -C≡CR⁹;
m is 0, 1 or 2;
n is 1 to 7;
p is 0 or 1;
G is oxygen or sulfur;
R¹⁰ is (C₁-C₁₀) straight or branched alkyl, (C₃-C₈) cycloalkyl, 2-, 3- or 4-pyridyl or 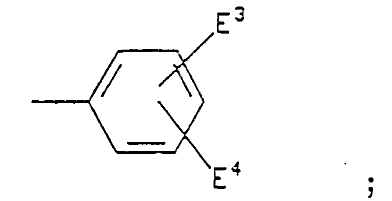
E¹, E², E³ and E⁴ are selected, independently, from hydrogen, halo, (C₁-C₃)alkyl, hydroxy, (C₁-C₃)alkoxy, -NO₂, -CF₃, -CN, -NH₂, (C₁-C₃)alkylamino and di[(C₁-C₃)alkyl]amino;
Het' and Het" are selected, independently, from 6 membered heterocyclic rings containing at least one nitrogen atom as part of the ring, optionally substituted with one substituent selected from (C₁-C₃)alkyl, halo, -OH, (C₁-C₃)alkoxy, -NH₂, (C₁-C₃)alkylamino and di-(C₁-C₃)alkylamino;
or a pharmaceutically acceptable salt thereof;for the manufacture of a medicament for the inhibition of abnormal cell growth in a mammal.
